(19) Europäisches Patentamt
European Patent Office
Office européen des brevets

(11) Veröffentlichungsnummer: **0 195 275 A2**

# (12) EUROPÄISCHE PATENTANMELDUNG

(21) Anmeldenummer: **86102254.9**

(51) Int. Cl.[4]: **C 07 D 251/16**

(22) Anmeldetag: **21.02.86**

(30) Priorität: **05.03.85 DE 3507749**

(43) Veröffentlichungstag der Anmeldung:
**24.09.86 Patentblatt 86/39**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fauss, Rudolf, Dr.**
**Gerstenkamp 10**
**D-5000 Köln 80(DE)**

(72) Erfinder: **Riebel, Hans-Jochem, Dr.**
**In der Beek 92**
**D-5600 Wuppertal 1(DE)**

## (54) Verfahren zur Herstellung von 4-Alkoxy-6-alkyl-2-cyanamino-1,3,5-triazinen.

(57) Man erhält in guten Ausbeuten 4-Alkoxy-6-alkyl-2-cyanamino-1,3,5-triazine der allgemeinen Formel (I)

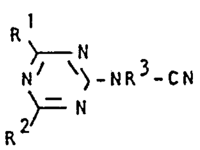
(I)

in welcher
$R^1$ für Alkyl steht,
$R^2$ für Alkoxy steht und
$R^3$ für Wasserstoff oder ein Aequivalent eines Alkali-oder Erdalkalimentallions steht, (welche als Zwischenprodukte zur Herstellung bekannter, herbizid wirksamer Guanidin-Derivate verwendet werden können), indem man entweder
(a) die neuen 6-Alkyl-4-chlor-2-cyanamino-1,3,5-triazine der Formel (II)

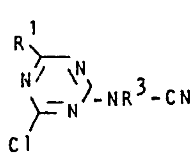
(II)

mit Alkoholaten der Formel (III)

$R^2Me$ (III)

in welcher
Me für ein Alkalimetallion steht, in Gegenwart von Verdünnungsmitteln umsetzt, oder indem man
(b) Verbindungen der Formel (Ia), die nach dem oben angegebenen Verfahren (a) hergestellt werden können,

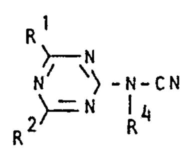
(Ia)

in welcher
$R^4$ für ein Äquivalent eines Alkali- oder Erdalkalimetallions steht,
in Gegenwart von Verdünnungsmitteln und in Gegenwart von Säuren zu den Verbindung der Formel (Ib)

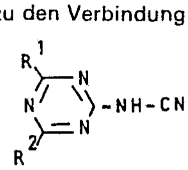
(Ib)

umsetzt.

Für die Herstellung der neuen Zwischenprodukte (II) wird ebenfalls ein Herstellungsverfahren, ausgehend von bekannten 2,4-Dichlor-6-alkly-1,3,5 triazinen, angegeben.

EP 0 195 275 A2

0195275

BAYER AKTIENGESELLSCHAFT 5090 Leverkusen-Bayerwerk

Konzernverwaltung RP

Patentabteilung Bi/mü /Ma

IVa/ZP

# Verfahren zur Herstellung von 4-Alkoxy-6-alkyl-2-cyanamino-1,3,5-triazinen

Die vorliegende Erfindung betrifft neue Verfahren zur Herstellung von 4-Alkoxy-6-alkyl-2-cyanamino-1,3,5-triazinen sowie neue Zwischenprodukte hierfür. Die Verfahrensprodukte sind zum Teil bekannt und können als Zwischenprodukte zur Herstellung von Herbiziden und Pflanzenwachstumsregulatoren verwendet werden.

Es ist bereits bekannt, daß 2-Cyanamino-1,3,5-triazine durch Umsetzung von Alkalimetall- oder Erdalkalimetall-Salzen von Cyanamid mit den entsprechenden 2-Halogen-1,3,5-triazinen erhalten werden (vgl. z.B. DE-OS 33 34 455 / EP-A- 121 082). Dieses Verfahren ist jedoch wegen des Fehlens geeigneter Ausgangsverbindungen bzw. wegen unbefriedigender Herstellungsmethoden hierfür nur sehr begrenzt anwendbar. Es besteht daher Bedarf an breiter anwendbaren Herstellungsverfahren für 4-Alkoxy-6-alkyl-2-cyanamino-1,3,5-triazine.

Es wurde nun gefunden, daß man 4-Alkoxy-6-alkyl-2-cyanamino-1,3,5-triazine der allgemeinen Formel (I)

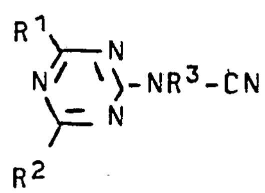

(I)

in welcher

$R^1$ für Alkyl steht,

$R^2$ für Alkoxy steht und

$R^3$ für Wasserstoff oder ein Aequivalent eines Alkali- oder Erdalkalimetallions steht,

erhält, wenn man

(a) 6 -Alkyl- 4-chlor-2-cyanamino-1,3,5-triazine der Formel (II)

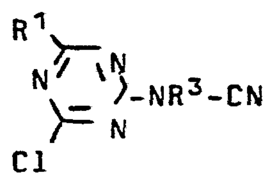

(II)

in welcher

$R^1$ und $R^3$ die oben angegebenen Bedeutungen haben,

mit Alkoholaten der Formel (III)

$$R^2Me \quad (III)$$

in welcher

$R^2$ die oben angegebene Bedeutung hat und

Me für ein Alkalimetallion steht,

in Gegenwart von Verdünnungsmitteln umsetzt,

oder wenn man

(b) Verbindungen der Formel (Ia), die nach dem oben angegebenen Verfahren (a) hergestellt werden können,

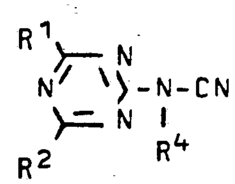

(Ia)

in welcher

$R^1$ und $R^2$ die oben angegebenen Bedeutungen haben und

$R^4$ für ein Aequivalent eines Alkali- oder Erdalkalimetallions steht,

in Gegenwart von Verdünnungsmitteln und in Gegenwart von Säuren zu den Verbindungen der Formel (I b)

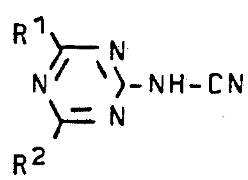

(Ib)

in welcher
$R^1$ und $R^2$ die oben angegebenen Bedeutungen haben,

umsetzt.

Ueberraschenderweise lassen sich mit Hilfe des erfindungsgemäßen Verfahrens die wertvollen Verbindungen der Formel (I) in glatten Reaktionen und in hohen Ausbeuten auf einfache Weise über die neuen Zwischenprodukte der Formel (II) herstellen.

Bevorzugt werden mit Hilfe des erfindungsgemäßen Verfahrens die Verbindungen der Formel (I) hergestellt, in welcher

$R^1$ für Alkyl mit 1 bis 6 Kohlenstoffatomen steht,

$R^2$ für Alkoxy mit 1 bis 6 Kohlenstoffatomen steht und

$R^3$ für Wasserstoff oder ein Aequivalent eines Natrium-, Kalium - oder Calciumions steht.

Besonders bevorzugt werden diejenigen Verbindungen der Formel (I) hergestellt, in welcher

$R^1$ für Methyl, Ethyl, n-Propyl, i-Propyl, n-Butyl, i-Butyl, sec.-Butyl oder tert.-Butyl steht,

$R^2$ für Methoxy, Ethoxy, n-Propoxy, i-Propoxy, n-Butoxy, i-Butoxy, sec.-Butoxy oder tert.-Butoxy steht und

$R^3$ für Wasserstoff oder ein Aequivalent eines Natrium - oder Kaliumions steht.

Ganz besonders bevorzugt werden diejenigen Verbindungen der Formel (I) hergestellt, in welcher

$R^1$ für Methyl, Ethyl, n-Propyl, i-Propyl oder n-Butyl steht,

$R^2$ für Methoxy, Ethoxy, n-Propoxy, i-Propoxy oder n-Butoxy steht und

$R^3$ für Wasserstoff oder ein Aequivalent eines Natrium- oder Kaliumions steht.

Verwendet man beispielsweise für das erfindungsgemäße Verfahren (a) 4-Chlor-2-cyanamino-6-methyl-1,3,5-triazin oder das entsprechende Natriumsalz und Natriummethanolat als Ausgangsstoffe, so können die Reaktionen durch die folgenden Formelschemata wiedergegeben werden:

(a)

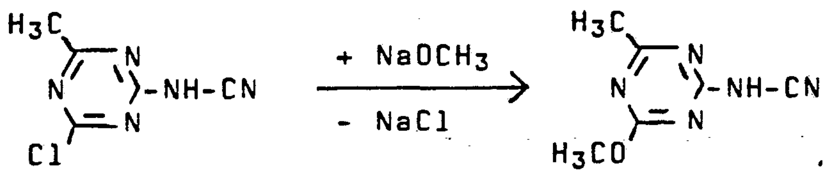


oder:

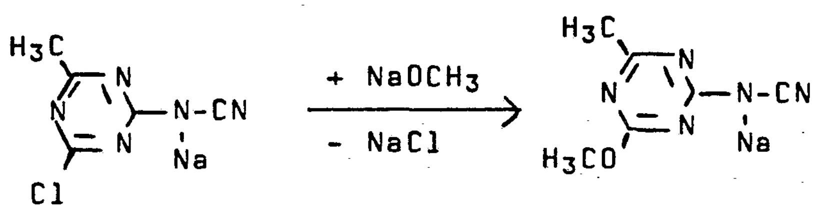

Verwendet man beispielsweise für das erfindungsgemäße Verfahren (b) das Kaliumsalz von 2-Cyanamino-6-methyl-4—ethoxy-1,3,5-triazin als Ausgangsstoff und Salzsäure als Mineralsäure, so kann die Reaktion durch das folgende Formelschema wiedergegeben werden:

(b)

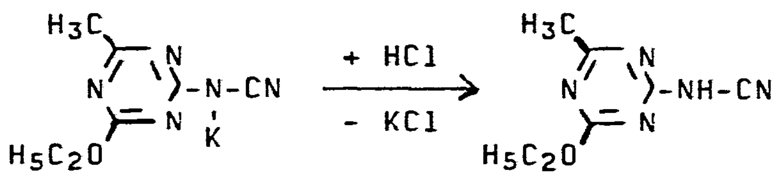


Die als Ausgangsstoffe für das erfindungsgemäße Verfahren (a) zu verwendenden 6-Alkyl-4-chlor-2-cyanamino-1,3,5-triazine sind durch die Formel (II) allgemein definiert. Bevorzugt sind diejenigen Verbindungen der Formel (II), in welcher

$R^1$ für Alkyl mit 1 bis 6 Kohlenstoffatomen steht, und

$R^3$ für Wasserstoff oder ein Aequivalent eines Natrium- , Kalium - oder Calciumions steht.

Besonders bevorzugt sind die Verbindungen der Formel (II), in welcher

$R^1$ für Methyl, Ethyl, n-Propyl,i-Propyl, n-Butyl, i-Butyl, sec.-Butyl oder tert.-Butyl steht und

$R^3$ für Wasserstoff oder ein Aequivalent eines Natrium- oder Kaliumions steht.

Ganz besonders bevorzugt sind die Verbindungen der Formel (II), in welcher

$R^1$ für Methyl, Ethyl, n-Propyl, i-Propyl oder n-Butyl steht und

$R^3$ für Wasserstoff oder ein Aequivalent eines Natrium- oder Kaliumions steht.

Als Beispiele für die Ausgangsstoffe der Formel (II) seien genannt:

6-Methyl- , 6-Ethyl- , 6-n-Propyl- , 6-i-Propyl- , 6-n-Butyl- , 6-i-Butyl-, 6-sec.-Butyl- und 6-tert.-Butyl-4-chlor-2-cyanamino-1,3,5-triazin und die entsprechenden Natrium- und Kaliumsalze.

Die Verbindungen der Formel (II) sind neu. Man erhält die Verbindungen der Formel (II), wenn man 2,4-Dichlor-6-alkyl-1,3,5-triazine der Formel (IV)

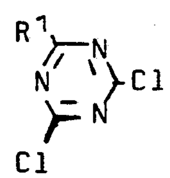

(IV)

in welcher

$R^1$ die oben angegebene Bedeutung hat,

mit Cyanamiden der Formel (V) ,

$$(R^4)_2N\text{-}CN \quad (V)$$

in welcher

$R^4$ die oben angegebene Bedeutung hat,

in Gegenwart von Wasser, bei einem pH-Wert zwischen 8,5 und 9,5 und bei Temperaturen zwischen -5°C und + 10°C zu den Verbindungen der Formel (IIa) ,

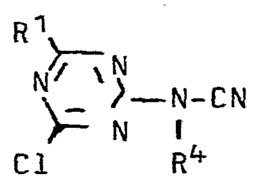

(IIa)

in welcher

$R^1$ und $R^4$ die oben angegebenen Bedeutungen haben,

umsetzt und diese Verbindungen der Formel (IIa) gegebenenfalls anschließend, gegebenenfalls nach ihrer Isolierung, in Gegenwart von Wasser und in Gegenwart von Säuren, wie z.B. Salzsäure, bei Temperaturen zwischen -10°C und + 20°C zu den Verbindungen der Formel (IIb)

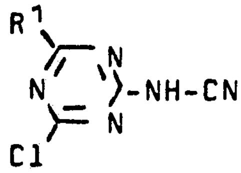 (II b)

in welcher

$R^1$ die oben angegebene Bedeutung hat,

umsetzt.

Die als Ausgangsstoffe für die Herstellung der Verbindundungen der Formel (IIa) bzw. (IIb) zu verwendenden 2,4-Dichlor -6-alkyl-1,3,5-triazine sind durch die Formel (IV) allgemein definiert. In dieser Formel steht $R^1$ bevorzugt für diejenigen Reste, welche oben im Rahmen der Substituentendefinition der Formel (II) vorzugsweise bzw. als insbesondere bevorzugt angegeben sind.

Als Beispiele für die Verbindungen der Formel (IV) seien genannt:

6-Methyl-, 6-Ethyl-, 6-n-Propyl-, 6-i-Propyl-, 6-n-Butyl-, 6-i-Butyl-, 6-sec.-Butyl- und 6-tert.-Butyl-2,4-dichlor-1,3,5-triazin.

Die Verbindungen der Formel (IV) sind bekannt (vgl. z.B. Helv. Chim. Acta 33 , 1365 (1950)).

Die weiterhin als Ausgangsstoffe für die Herstellung der Verbindungen der Formel (IIa) bzw. (IIb) zu verwendenden Cyanamide sind durch die Formel (V) allgemein definiert. In dieser Formel steht $R^4$ vorzugsweise für ein Aequivalent eines Natrium -, Kalium - oder Calciumions.

Als Beispiele für die Verbindungen der Formel (V) seien genannt:

Di-Natrium-cyanamid , Di-Kalium-cyanamid und Calciumcyanamid.

Die Verbindungen der Formel (V) sind allgemein bekannte Verbindungen der organischen Chemie.

Die weiterhin als Ausgangsstoffe für das erfindungsgemäße Verfahren (a) zu verwendenden Alkoholate sind durch die Formel (III) allgemein definiert. In dieser Formel hat $R^2$ vorzugsweise diejenigen Bedeutungen, welche oben im Rahmen der Substituentendefinition der Formel (I) vorzugsweise bzw. als insbesondere bevorzugt angegeben sind. Me steht in dieser Formel vorzugsweise für ein Natrium- oder Kaliumion.

Als Beispiele für die Verbindungen der Formel (III) seien genannt:

Natrium-methylat, -ethylat, -n-propylat, -i-propylat, -n-butylat, -i-butylat, -sec-butylat und tert.-butylat und die entsprechenden Kalium-Derivate.

Die Verbindungen der Formel (III) sind allgemein bekannte Verbindungen der organischen Chemie.

Das erfindungsgemäße Verfahren (a) wird in Gegenwart von Verdünnungsmitteln durchgeführt.

Hierzu gehören insbesondere aliphatische und aromatische Kohlenwasserstoffe, wie Pentan, Hexan, Heptan, Cyclohexan, Petrolether, Benzin, Ligroin, Benzol, Toluol, Xylol, Alkohole, wie Methanol, Ethanol, n-Propanol, i-Propanol, n-Butanol, i-Butanol, sec.-Butanol und tert.-Butanol. Bevorzugt werden die entsprechenden Alkohole der eingesetzten Alkoholate der Formel (III) verwendet.

Das erfindungsgemäße Verfahren (a) wird im allgemeinen bei Temperaturen zwischen 0°C und 50°C, vorzugsweise zwischen 0°C und 40°C durchgeführt. Die Umsetzungen werden im allgemeinen bei Normaldruck durchgeführt.

Bei der Durchführung des erfindungsgemäßen Verfahrens (a) setzt man auf 1 Mol der Verbindung der Formel (IIa) 1,0 bis 1,4 Mol, vorzugsweise 1 bis 1,2 Mol Alkoholat der Formel (III) ein oder man setzt auf 1 Mol der Verbindung der Formel (IIb) 2,0 bis 2,5 Mol, vorzugsweise 2,0 bis 2,3 Mol Alkoholat der Formel (III) ein.
Die Aufarbeitung der Verbindungen der Formel (I) geschieht nach üblichen Methoden. Nach beendeter Zugabe der Ausgangsstoffe wird noch kurze Zeit oder auch mehrere Stunden bei 15°C bis 25°C nachgerührt. Anschließend wird für den Fall, daß Verbindungen der Formel (Ia) hergestellt

werden, gegebenenfalls mit Alkohol verdünnt und im Vakuum bei Temperaturen unter 50°C eingeengt und für den Fall, daß Verbindungen der Formel (Ib) hergestellt werden, mit Wasser versetzt und mit einer Mineralsäure, wie z.B. Salzsäure angesäuert. Die Verbindungen der Formel (I) fallen im allgemeinen in kristalliner Form an.

Die als Ausgangsstoffe für das erfindungsgemäße Verfahren (b) zu verwendenden Verbindungen sind durch die Formel (Ia) allgemein definiert. In dieser Formel stehen $R^1$ und $R^2$ für diejenigen Reste, welche oben im Rahmen der Substituentendefinition der Formel (I) vorzugsweise bzw. als insbesondere bevorzugt angegeben sind. In dieser Formel steht $R^4$ vorzugsweise für ein Aequivalent eines Natrium -, Kalium - oder Calciumions.

Als Beispiele für die Verbindungen der Formel (Ia) seien genannt:

Die Natrium-, Kalium- und Calciumsalze von

6-Methyl-4-methoxy-, 6-Methyl-4-ethoxy-, 6-Methyl-4-n-propoxy-, 6-Methyl-4-i-propoxy-, 6-Methyl-4-n-butoxy-, 6-Methyl-4-i-butoxy-, 6-Methyl-4-sec.-butoxy-, 6-Methyl-4-tert.-butoxy-, 6-Ethyl-4-methoxy-, 6-Ethyl-4-ethoxy-,6-Ethyl-4-n-propoxy-, 6-Ethyl-4-i-propoxy-, 6-Ethyl-4-n-butoxy-, 6-Ethyl-4-i-butoxy-, 6-Ethyl-4-sec.-butoxy-, 6-Ethyl-4-tert.-butoxy-, 6-n-Propyl-4-methoxy-, 6-n-Propyl-4-ethoxy-, 6-n-Propyl-4-n-propoxy-, 6-n-Propyl-4-propoxy-, 6-n-Propyl-4-n-butoxy-, 6-n-Propyl-4-i-butoxy-,

6-n-Propyl-4-sec.-butoxy-, 6-n-Propyl-4-tert.-butoxy-, 6-i-Propyl-4-methoxy-, 6-i-Propyl-4-ethoxy-, 6-i-Propyl-4-n-propoxy-, 6-i-Propyl-4-i-propoxy-, 6-i-Propyl-4-n-butoxy-, 6-i-Propyl-4-i-butoxy-, 6-i-Propyl-4-sec.-butoxy-, 6-i-Propyl-4-tert.-butoxy-, 6-n-Butyl-4-methoxy-, 6-n-Butyl-4-ethoxy-, 6-n-Butyl-4-n-propoxy-, 6-n-Butyl-4-i-propoxy-, 6-n-Butyl-4-n-butoxy-, 6-n-Butyl-4-i-butoxy-, 6-n-Butyl-4-sec.-butoxy-, 6-n-Butyl-4-tert.-butoxy-, 6-i-Butyl-4-methoxy-,6-i-Butyl-4-ethoxy-, 6-i-Butyl-4-n-propoxy-, 6-i-Butyl-4-i-propoxy-, 6-i-Butyl-4-n-butoxy-, 6-i-Butyl-4-i-butoxy-, 6-i-Butyl-4-sec.-butoxy-, 6-i-Butyl-4-tert.-butoxy-, 6-sec.-Butyl-4-methoxy-, 6-sec.-Butyl-4-ethoxy-, 6-sec.-Butyl-4-n-propoxy-, 6-sec.-Butyl-4-i-propoxy-, 6-sec.-Butyl-4-n-butoxy-, 6-sec.-Butyl-4-i-butoxy-, 6-sec.-Butyl-4-sec.-butoxy-, 6-sec.-Butyl-4-tert.-butoxy-, 6-tert.-Butyl-4-methoxy-, 6-tert.-Butyl-4-ethoxy-, 6-tert.-Butyl-4-n-propoxy-, 6-tert.-Butyl-4-i-propoxy-, 6-tert.-Butyl-4-n-butoxy-, 6-tert.-Butyl-4-i-butoxy-, 6-tert.-Butyl-4-sec.-butoxy- und 6-tert.-Butyl-4-tert.-butoxy-2-cyanamino-1,3,5-triazin.

Die Verbindungen der Formel (Ia) lassen sich nach dem erfindungsgemäßen Verfahren (a) herstellen.

Das erfindungsgemäße Verfahren (b) wird vorzugsweise in Gegenwart von Wasser als Verdünnungsmittel durchgeführt.

Das erfindungsgemäße Verfahren (b) wird in Gegenwart von Säuren durchgeführt. Besonders bewährt haben sich Salzsäure, Bromwasserstoffsäure, Schwefelsäure, Phosphorsäure und Essigsäure. Bevorzugt wird Salzsäure verwendet.

Das erfindungsgemäße Verfahren (b) wird im allgemeinen bei Temperaturen zwischen -10°C und + 25°C, vorzugsweise zwischen -5°C und + 20°C durchgeführt. Die Umsetzungen werden im allgemeinen bei Normaldruck durchgeführt.
Bei der Durchführung des erfindungsgemäßen Verfahrens (b) setzt man auf 1 Mol der Verbindung der Formel (Ia) 1 bis 3 Mol, vorzugsweise 1,8 bis 2,3 Mol Säure ein.
Die Aufarbeitung erfolgt nach üblichen Methoden.

Die nach den erfindungsgemäßen Verfahren herzustellenden 4-Alkoxy-6-alkyl-2-cyanamino-1,3,5-triazine können als Zwischenprodukte für die Herstellung von Guanidin-Derivaten, die als Herbizide und Pflanzenwuchsregulatoren wirksam sind, eingesetzt werden (vgl. EP-OS 121 082).

Herstellungsbeispiele

Beispiel 1

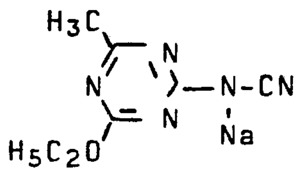

(Verfahren (a) )

Zu einer Lösung von 29 g (0,15 Mol) des Natriumsalzes von 4-Chlor-2-cyanamino-6-methyl-1,3,5-triazin in 500 ml Ethanol tropft man eine Lösung von 3,7 g (0,16 Mol) Natrium in 100 ml Ethanol in der Weise, daß eine Reaktionstemperatur von 30°C nicht überschritten wird. Anschliessend wird bei 20°C 3 Stunden nachgerührt, mit 400 ml Ethanol verdünnt und filtriert. Das Filtrat wird im Vakuum eingeengt, wobei eine Temperatur von 45°C nicht überschritten wird.

Man erhält so 29,9 g (99 % der Theorie) des Natriumsalzes von 2-Cyanamino-4-ethoxy-6-methyl-1,3,5-triazin vom Schmelzpunkt 200°C (Zers.)

Beispiel 2

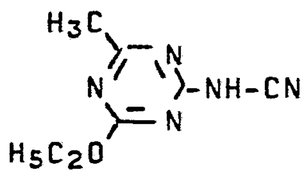

(Verfahren (a) )

Zu einer Lösung von 2,5 g (0,11 Mol) Natrium in 100 ml Ethanol gibt man portionsweise 8,5 g (0,05 Mol) 4-Chlor-2-cyanamino-6-methyl-1,3,5-triazin in der Weise, daß eine Temperatur von 35°C nicht überschritten wird. Anschließend wird 1 Stunde bei 20°C nachgerührt und im Vakuum in der Weise eingeengt, daß eine Badtemperatur von 50°C nicht überschritten wird. Der Rückstand wird in 50 ml Wasser gelöst und mit 6 ml konzentrierter Salzsäure angesäuert. Die entstandenen Kristalle werden abgesaugt und getrocknet.

Man erhält 8,1 g (91 % der Theorie) 2-Cyanamino-4-ethoxy-6-methyl-1,3,5-triazin vom Schmelzpunkt 195°C (Zers.).

Beispiel 3

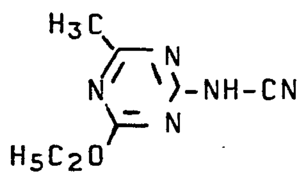

(Verfahren (b) )

Zu einer Lösung von 30,1 g (0,15 Mol) des Natriumsalzes von 2-Cyanamino-4-ethoxy-6-methyl-1,3,5-triazin in 300 ml Wasser werden bei 0°C bis 10°C 50 ml 20 %ige Salzsäure gegeben und 15 Minuten bei 10°C nachgerührt. Der Niederschlag wird abgesaugt und getrocknet.

Man erhält 26,5 g (98 % der Theorie) 2-Cyanamino-4-ethoxy-6-methyl-1,3,5-triazin vom Schmelzpunkt 195°C (Zers.).

Analog Beispiel 1 bis 3 bzw. Verfahren (a) und (b) können die folgenden Verbindungen hergestellt werden:

Beispiel 4

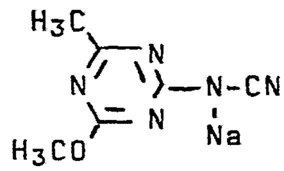

Fp : 220°C (Zers.)

Beispiel 5

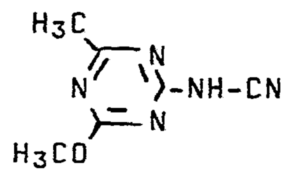

Fp : 184°C (Zers.)

Herstellung von Ausgangsstoffen der Formel (II)

Beispiel (II-1)

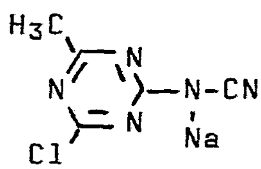

Zu einer Suspension von 8,2 g (0,05 Mol) 2,4-Dichlor-6-methyl-1,3,5-triazin in 100 ml Eiswasser tropft man bei einer Temperatur von 0°C bis 5°C 4,5 g (0,05 Mol) Dinatriumcyanamid in 50 ml Wasser in der Weise, daß ein pH Wert von 9,5 nicht überschritten wird. Anschließend wird noch 1 Stunde bei 20°C nachgerührt, mit 40 g Natriumchlorid versetzt und ca. 0,5 bis 1 Stunde bei 20°C nachgerührt.

Nach dem Absaugen und Trocknen erhält man 9,1 g (95 % der Theorie) des Natriumsalzes von 4-Chlor-2-cyanamino-6-methyl-1,3,5-triazin vom Schmelzpunkt 190°C (Zers.).

Beispiel (II-2)

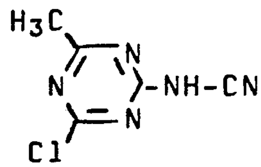

Zu einer Suspension von 2 g (0,01 Mol) des Natriumsalzes von 4-Chlor-2-cyanamino-6-methyl-1,3,5-triazin in 50 ml Wasser wird bei 0°C bis 10°C 1 ml konzentrierter Salzsäure gegeben. Der entstehende Niederschlag wird abgesaugt und getrocknet.

Man erhält so 1,3 g (77 % der Theorie) 4-Chlor-2-cyanamino-6-methyl-1,3,5-triazin vom Schmelzpunkt 105°C (Zers.).

Patentansprüche

1) Verfahren zur Herstellung von 4-Alkoxy-6-alkyl-2-cyanamino-1,3,5-triazinen der allgemeinen Formel (I)

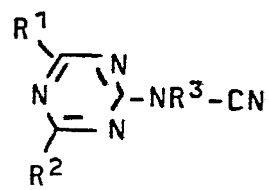

(I)

in welcher

$R^1$ für Alkyl steht,

$R^2$ für Alkoxy steht und

$R^3$ für Wasserstoff oder ein Äquivalent eines Alkali- oder Erdalkalimetallions steht,

dadurch gekennzeichnet, daß man

(a) 6-Alkyl-4-chlor-2-cyanamino-1,3,5-triazine der Formel (II)

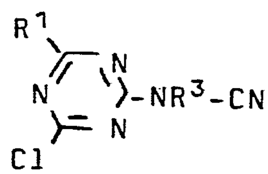

(II)

in welcher

$R^1$ und $R^3$ die oben angegebenen Bedeutungen haben,

mit Alkoholaten der Formel (III)

$$R^2Me \quad (III)$$

in welcher

$R^2$ die oben angegebene Bedeutung hat und

Me für ein Alkalimetallion steht,

in Gegenwart von Verdünnungsmitteln umsetzt,

oder daß man

(b) Verbindungen der Formel (Ia), die nach dem oben angegebenen Verfahren hergestellt werden können,

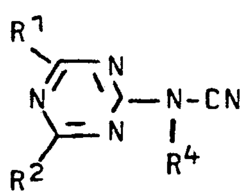 (Ia)

in welcher

$R^1$ und $R^2$ die oben angegebenen Bedeutungen haben und

$R^4$ für ein Aequivalent eines Alkali- oder Erdalkalimetallions steht,

in Gegenwart von Verdünnungsmitteln und in Gegenwart von Säuren zu den Verbindungen der Formel (Ib)

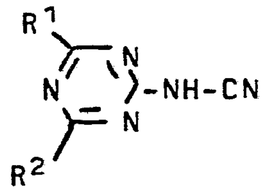

(Ib)

in welcher

$R^1$ und $R^2$ die oben angegebenen Bedeutungen haben,

umsetzt.

2) 6-Alkyl-4-chlor-2-cyanamino-1,3,5-triazine der Formel (II)

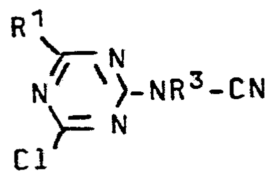

(II)

in welcher

$R^1$ für Alkyl steht und

$R^3$ für Wasserstoff oder ein Äquivalent eines Alkali- oder Erdalkalimetallions steht.

3) Verfahren zur Herstellung von 6-Alkyl-4-chlor-2-cyan-amino-1,3,5-triazinen der Formel (II), gemäß Anspruch 2, dadurch gekennzeichnet, daß man 2,4-Dichlor-6-alkyl-1,3,5-triazine der Formel (IV)

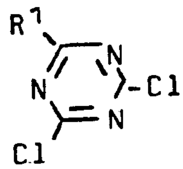

(IV)

in welcher

$R^1$ die oben angegebene Bedeutung hat,

mit Cyanamiden der Formel (V),

$(R^4)_2N-CN$ (V)

in welcher

$R^4$ die oben angegebene Bedeutung hat,

in Gegenwart von Wasser, bei einem pH-Wert zwischen 8,5 und 9,5 und bei Temperaturen zwischen -5°C und +10°C zu den Verbindungen der Formel (IIa),

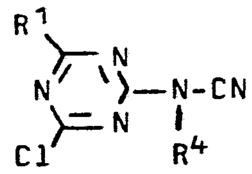

(IIa)

in welcher

$R^1$ und $R^4$ die oben angegebenen Bedeutungen haben,

umsetzt

und diese Verbindungen der Formel (IIa) gegebenenfalls anschließend, gegebenenfalls nach ihrer Isolierung, in Gegenwart von Wasser und in Gegenwart von Säure, wie z.B. Salzsäure, bei Temperaturen zwischen -10°C und + 20°C zu den Verbindungen der Formel (IIb)

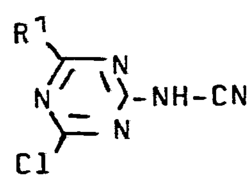

(IIb)

in welcher

$R^1$ die oben angegebene Bedeutung haben,

umsetzt.